(19) Europäisches Patentamt
European Patent Office
Office européen des brevets

(11) Veröffentlichungsnummer: **0 022 531**
**B1**

(12) # EUROPÄISCHE PATENTSCHRIFT

(45) Veröffentlichungstag der Patentschrift: **02.03.83**

(51) Int. Cl.³: **C 07 D 249/08**, A 01 N 43/64

(21) Anmeldenummer: **80103845.6**

(22) Anmeldetag: **07.07.80**

(54) **1-(2,4-Dichlorphenyl)-1-(2,6-dihalogenbenzylmercapto)-2-(1,2,4-triazol-1-yl)-ethane, Verfahren zu ihrer Herstellung und ihre Verwendung als Fungizide.**

(30) Priorität: **17.07.79 DE 2928768**

(43) Veröffentlichungstag der Anmeldung: **21.01.81 Patentblatt 81/3**

(45) Bekanntmachung des Hinweises auf die Patenterteilung: **02.03.83 Patentblatt 83/9**

(84) Benannte Vertragsstaaten: **AT BE CH DE FR GB IT LI NL**

(56) Entgegenhaltungen:
**EP-A-0 000 112**
**DE-A-2 645 496**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal-1 (DE)**
Erfinder: **Büchel, Karl Heinz, Dr., Bergerheide 62, D-5600 Wuppertal-1 (DE)**
Erfinder: **Timmler, Helmut, Dr., Tersteegenweg 16, D-5600 Wuppertal-1 (DE)**
Erfinder: **Brandes, Wilhelm, Dr., Eichendorffstrasse 3, D-5623 Leichlingen (DE)**

EP 0 022 531 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 1-(2,4-Dichlorphenyl)-1-(2,6-dihalogenbenzylmercapto)-2-(1,2,4-triazol-1-yl)-ethane, Verfahren zu ihrer Herstellung und ihre Verwendung als Fungizide

Die vorliegende Erfindung betrifft neue 1-(2,4-Dichlorphenyl)-1-(2,6-dihalogenbenzylmercapto)-2-(1,2,4-triazol-1-yl)-ethane, mehrere Verfahren zu ihrer Herstellung und ihre Verwendung als Fungizide.

Es ist bereits bekanntgeworden, daß bestimmte substituierte 1-Benzylmercapto-1-phenyl-2-(1,2,4-triazol-1-yl)-ethane, wie beispielsweise 1-(2,4-Dichlorphenyl)-1-(2,4-dichlorbenzylmercapto)-2-(1,2,4-triazol-1-yl)-ethan, gute fungizide Eigenschaften aufweisen (vergleiche DE-OS-2 645 496 und DE-OS 2 724 684). Deren Wirkung ist jedoch in bestimmten Indikationsbereichen, insbesondere bei niedrigen Aufwandmengen und -konzentrationen, nicht immer voll befriedigend.

Es wurden neue 1-(2,4-Dichlorphenyl)-1-(2,6-dihalogenbenzylmercapto)-2-(1,2,4-triazol-1-yl)-ethane der allgemeinen Formel

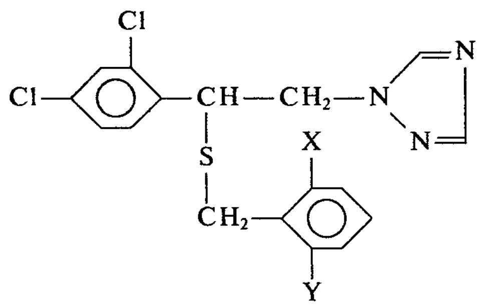

(I)

in welcher

X und Y gleich oder verschieden sind und für Halogen stehen,

und deren physiologisch verträglichen Säureadditions-Salze gefunden.

Weiterhin wurde gefunden, daß man die 1-(2,4-Dichlorphenyl)-1-(2,6-dihalogenbenzylmercapto)-2-(1,2,4-triazol-1-yl)-ethane der Formel (I) erhält, wenn man

a) 1-(2,4-Dichlorphenyl)-1-halogen-2-(1,2,4-triazol-1-yl)-ethane der Formel

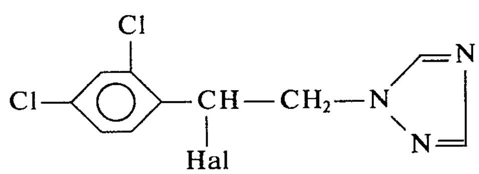

(II)

in welcher

Hal für Halogen steht,

mit Mercaptanen der Formel

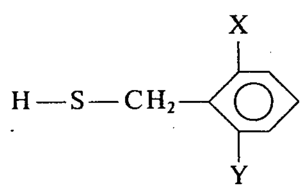

(III)

in welcher

X und Y die oben angegebene Bedeutung haben,

gegebenenfalls in Gegenwart eines Verdünnungsmittels und eines Säurebindemittels umsetzt, wobei die Mercaptane der Formel (III) vorzugsweise in-situ eingesetzt werden, oder

b) 1-(2,4-Dichlorphenyl)-2-(1,2,4-triazol-1-yl)-ethan-1-thiol der Formel

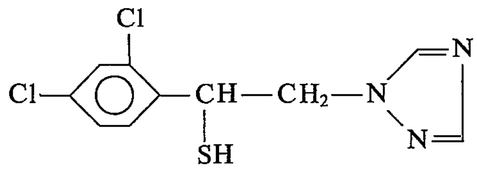

(IV)

mit 2,6-Dihalogenbenzyl-Derivaten der Formel

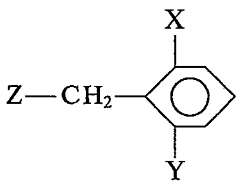

(V)

in welcher

X und Y die oben angegebene Bedeutung haben und
Z für Halogen, und die Gruppierungen $-O-SO_2-R^1$ und $-N(R^2)_3^{(+)}$ steht, wobei
$R^1$ für Alkyl oder gegebenenfalls substituiertes Phenyl steht und
$R^2$ für Alkyl steht,

in Gegenwart eines Verdünnungsmittels und in Gegenwart einer Base umsetzt.
An die so erhaltenen Verbindungen der Formel (I) kann gegebenenfalls anschließend eine Säure addiert werden.

Weiterhin wurde gefunden, daß die neuen 1-(2,4-Dichlorphenyl)-1-(2,6-dihalogenbenzylmercapto)-2-(1,2,4-triazol-1-yl)-ethane starke fungizide Eigenschaften aufweisen. Dabei zeigen überraschenderweise die erfindungsgemäßen Verbindungen eine erheblich höhere Wirkung als das aus dem Stand der Technik bekannte 1-(2,4-Dichlorphenyl)-1-(2,4-dichlorbenzylmercapto)-2-(1,2,4-triazol-1-yl)-ethan, welches chemisch und wirkungsmäßig die naheliegendste Verbindung ist. Die erfindungsgemäßen Stoffe stellen somit eine Bereicherung der Technik dar.

Die erfindungsgemäßen 1-(2,4-Dichlorphenyl)-1-(2,6-dihalogenbenzylmercapto)-2-(1,2,4-triazol-1-yl)-ethane sind durch die Formel (I) allgemein definiert. In dieser Formel sind X und Y gleich oder verschieden und stehen vorzugsweise für Chlor oder Fluor. An Beispielen für Verbindungen der Formel (I) sind zu nennen:
1-(2,4-Dichlorphenyl)-1-(2,6-dichlorbenzylmercapto)-2-(1,2,4-triazol-1-yl)-ethan,
1-(2,4-Dichlorphenyl)-1-(2-chlor-6-fluorbenzylmercapto)-2-(1,2,4-triazol-1-yl)-ethan und
1-(2,4-Dichlorphenyl)-1-(2,6-difluorbenzylmercapto)-2-(1,2,4-triazol-1-yl)-ethan und
deren Salze.

Verwendet man beispielsweise 1-Chlor-1-(2,4-dichlorphenyl)-2-(1,2,4-triazol-1-yl)-ethan und 2,6-Dichlorbenzylmercaptan als Ausgangsstoffe, so kann der Reaktionsablauf durch das folgende Formelschema wiedergegeben werden (Verfahren a):

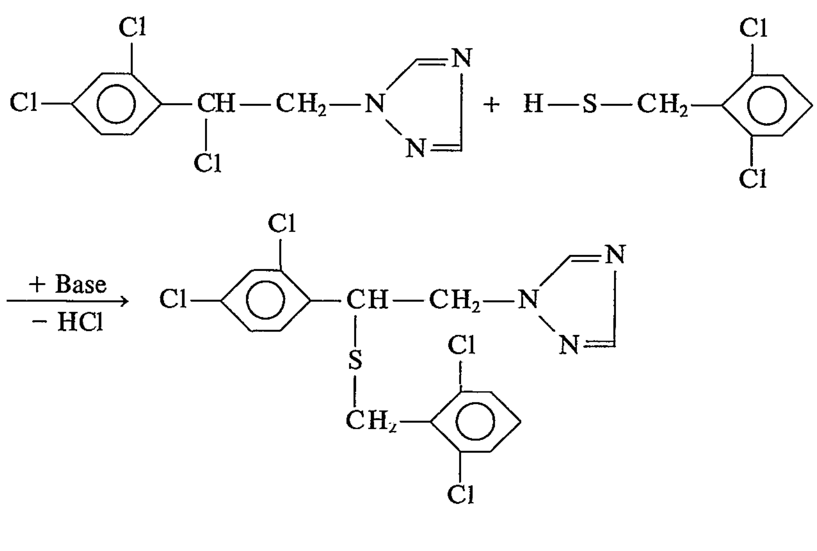

Verwendet man beispielsweise 1-Chlor-1-(2,4-dichlorphenyl)-2-(1,2,4-triazol-1-yl)-ethan, 2,6-Dichlorbenzylchlorid und Thioharnstoff als Ausgangsstoffe, so kann der Reaktionsablauf durch das folgende Formelschema wiedergegeben werden (Verfahren (a) ohne Isolierung des Zwischenproduktes):

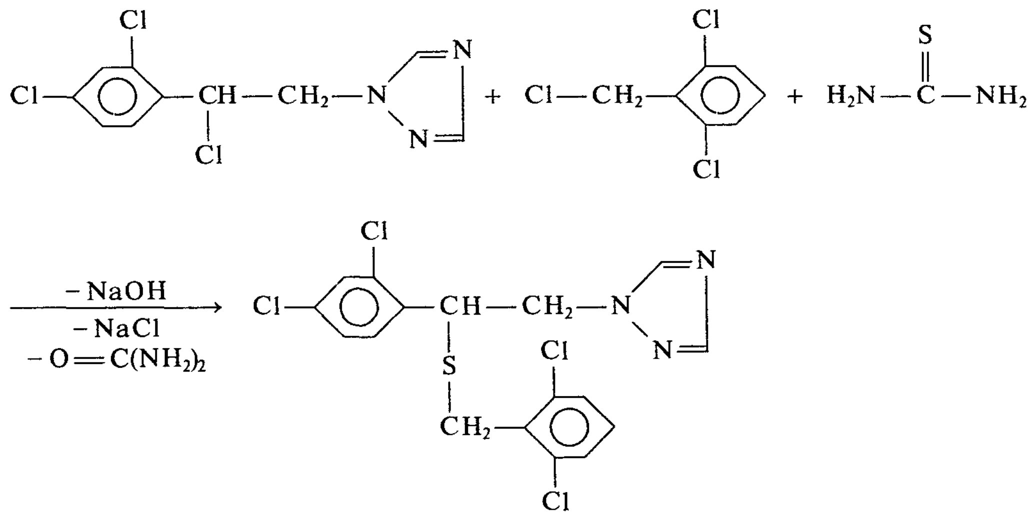


Verwendet man beispielsweise 1-(2,4-Dichlorphenyl)-2-(1,2,4-triazol-1-yl)-ethan-1-thiol und 2,6-Dichlorbenzylchlorid als Ausgangsstoffe, so kann der Reaktionsablauf durch das folgende Formelschema wiedergegeben werden (Verfahren b):

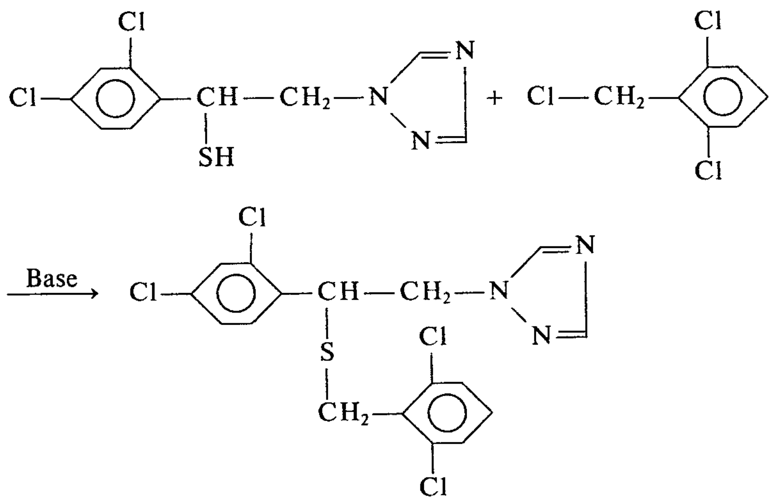


Die bei der Durchführung des erfindungsgemäßen Verfahrens (a) als Ausgangsstoffe zu verwendenden 1-(2,4-Dichlorphenyl)-1-halogen-2-(1,2,4-triazol-1-yl)-ethane sind durch die Formel (II) allgemein definiert. In dieser Formel steht Hal vorzugsweise für Chlor oder Brom.

Die 1-(2,4-Dichlorphenyl)-1-halogen-2-(1,2,4-triazol-1-yl)-ethane der Formel (II) sind bekannt (vergleiche DE-OS 2 547 954). Sie werden erhalten, indem man das entsprechende bekannte 1-(2,4-Dichlorphenyl)-1-hydroxy-2-(1,2,4-triazol-1-yl)-ethan (vergleiche DE-OS 2 431 407) mit einem halogenabspaltenden Mittel, beispielsweise Thionylchlorid, gegebenenfalls in Gegenwart eines Verdünnungsmittels, wie beispielsweise Chloroform, bei Temperaturen zwischen 20 und 100°C umsetzt.

Die außerdem für das erfindungsgemäße Verfahren (a) als Ausgangsstoffe zu verwendenden Mercaptane sind durch die Formel (III) allgemein definiert. In dieser Formel stehen X und Y vorzugsweise für diejenigen Reste, die bereits im Zusammenhang mit der Beschreibung der erfindungsgemäßen Stoffe der Formel (I) vorzugsweise für diese Substituenten genannt wurden.

Die Mercaptane der Formel (III) sind allgemein bekannte Verbindungen der organischen Chemie und werden in allgemein üblicher Weise erhalten, indem man bekannte 2,6-Dihalogenbenzylhalogenide der Formel

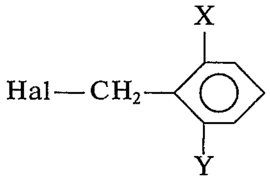

(VI)

in welcher

Hal, X und Y die oben angegebene Bedeutung haben,

mit Thioharnstoff in Gegenwart einer starken Base, wie insbesondere Natronlauge, und in Gegenwart eines Lösungsmittels, wie beispielsweise Ethanol erhitzt. Die dabei entstehenden Mercaptane können ohne Isolierung in einer Eintopfreaktion direkt mit den Verbindungen der Formel (II) umgesetzt werden (vergleiche auch die Herstellungsbeispiele).

Das bei der Durchführung des erfindungsgemäßen Verfahrens (b) als Ausgangsstoff zu verwendende 1-(2,4-Dichlorphenyl)-2-(1,2,4-triazol-1-yl)-ethan-1-thiol der Formel (IV) ist beschrieben (vergleiche DE-OS-2 645 496); zur Herstellung siehe die Herstellungsbeispiele.

Die außerdem für das erfindungsgemäße Verfahren (b) als Ausgangsstoffe zu verwendenden 2,6-Dihalogenbenzyl-Derivate sind durch die Formel (V) allgemein definiert. In dieser Formel stehen X und Y vorzugsweise für diejenigen Reste, die bereits im Zusammenhang mit der Beschreibung der erfindungsgemäßen Stoffe der Formel (I) vorzugsweise für diese Substituenten genannt wurden. Z steht vorzugsweise für Chlor, Brom, Jod sowie die Gruppierungen $-O-SO_2-R^1$ und $-N(R^2)_3^{(+)}$, wobei $R^1$ vorzugsweise für Alkyl mit 1 bis 4 Kohlenstoffatomen oder gegebenenfalls durch Alkyl mit 1 bis 2 Kohlenstoffatomen, Chlor, Brom, Alkoxy mit 1 bis 2 Kohlenstoffatomen oder Nitro substituiertes Phenyl steht, und $R^2$ vorzugsweise für Alkyl mit 1 bis 2 Kohlenstoffatomen steht.

Die 2,6-Dihalogenbenzyl-Derivate der Formel (V) sind allgemein bekannte Verbindungen der organischen Chemie.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Sorbinsäure, Milchsäure, sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Für die erfindungsgemäße Umsetzung gemäß Verfahren (a) kommen als Verdünnungsmittel vorzugsweise inerte organische Lösungsmittel bzw. aprotische Lösungsmittel infrage. Hierzu gehören vorzugsweise Ketone, wie Diethylketon, insbesondere Aceton und Methylethylketon; Alkohole, wie Ethanol oder Isopropanol; Nitrile, wie Propionitril, insbesondere Acetonitril; Kohlenwasserstoffe, wie Ligroin, Petrolether, Benzol, Toluol, Xylol; sowie Formamide, wie insbesondere Dimethylformamid.

Die erfindungsgemäße Umsetzung gemäß Verfahren (a) wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, Dimethylbenzylamin oder Dimethylcyclohexylamin; oder wie Pyridin und Diazabicyclooctan sowie Alkalialkoholate, beispielsweise Natriummethylat, Kaliumethylat oder wie Alkalihydroxide, beispielsweise Natriumhydroxid und Kaliumhydroxid.

Die Reaktionstemperaturen können bei der Durchführung des Verfahrens (a) in einem größeren Bereich variiert werden. Im allgemeinen arbeitet man zwischen 20 und 150°C, vorzugsweise bei der Siedetemperatur des Lösungsmittels, beispielsweise zwischen 60 und 100°C.

Bei der Durchführung des erfindungsgemäßen Verfahrens (a) setzt man auf 1 Mol der Verbindungen der Formel (II) vorzugsweise etwa 1 bis 2 Mol des Mercaptans der Formel (III) und etwa 1,5 bis 2,5 Mol Säurebinder ein. Zur Isolierung der Verbindungen der Formel (I) wird das Reaktionsgemisch filtriert, das Filtrat eingeengt und der Rückstand mit wäßriger Base digeriert. Anschließend wird mit einem organischen Solvents ausgeschüttelt und das Reaktionsprodukt in üblicher Weise isoliert. Die Reaktionsprodukte können gegebenenfalls durch Umkristallisation gereinigt werden.

In einer bevorzugten Durchführung des erfindungsgemäßen Verfahrens (a) arbeitet man in einer Eintopfreaktion, d. h. ohne Isolierung der Mercaptane der Formel (III). Dabei setzt man auf 1 Mol der Verbindungen der Formel (II) vorzugsweise 1,2 bis 2 Mol Benzylhalogenid der Formel (IV), 1,2 bis 2 Mol

Thioharnstoff und 2 bis 3 Mol Base ein. Die Isolierung der Verbindungen der Formel (I) erfolgt in üblicher Weise.

Für die erfindungsgemäße Umsetzung gemäß Verfahren (b) kommen als Verdünnungsmittel protische und aprotische Lösungsmittel infrage, insbesondere solche, in denen $S_N1$- und $S_N2$-Reaktionen durchgeführt werden. Hierzu gehöhren beispielsweise Wasser, Alkohole, Carbonsäuren, Aceton, Acetonitril, Dimethylformamid, Dimethylacetamid, Dimethylsulfoxid, Tetramethylharnstoff oder aromatische Kohlenwasserstoffe, wie Toluol.

Die erfindungsgemäße Umsetzung gemäß Verfahren (b) wird in Gegenwart einer Base durchgeführt. Hierzu gehören alle üblicherweise verwendbaren organischen und insbesondere anorganischen Basen, wie beispielsweise Natrium- und Kaliumhydroxid oder Natrium- und Kaliumcarbonat.

Die Reaktionstemperaturen können bei der Durchführung des erfindungsgemäßen Verfahrens (b) in einem größeren Bereich variiert werden. Im allgemeinen arbeitet man zwischen 20 und 150°C, vorzugsweise zwischen 20 und 100°C, bzw. bei der Siedetemperatur des jeweiligen Lösungsmittels.

Bei der Durchführung des erfindungsgemäßen Verfahrens (b) setzt man vorzugsweise auf 1 Mol der Verbindung der Formel (IV) 1 bis 3 Mol 2,6-Dihalogenbenzyl-Derivat der Formel (V) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

Die erfindungsgemäßen Wirkstoffe weisen eine starke fungizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung echter Mehltauarten, wie gegen den Erreger des echten Gurkenmehltaus (Erysiphe cichoracearum) und des echten Apfelmehltaus (Podosphaera leucotricha), eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Beispiel A

### Erysiphe-Test (Gurken)/Protektiv

Lösungsmittel: 4,7 Gewichtsteile Aceton
Emulgator: 0,3 Gewichtsteile Alkyl-aryl-polyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24°C und bei einer relativen Luftfeuchtigkeit von ca. 75% im Gewächshaus aufgestellt.

Nach 12 Stunden wird der Befall der Gurkenpflanzen bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse gehen aus der nachfolgenden Tabelle hervor.

Tabelle A

Erysiphe-Test (Gurken)/Protektiv

| Wirkstoff | Befall in % bei einer Wirkstoff-konzentration von 0,0005% |
|---|---|
| 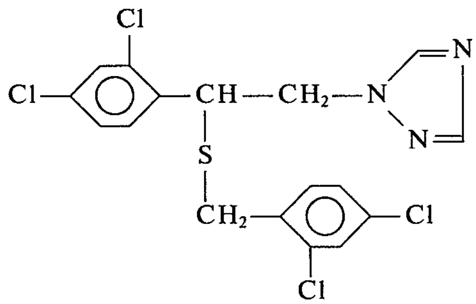 $\times HNO_3$ (bekannt) | 12 |
| 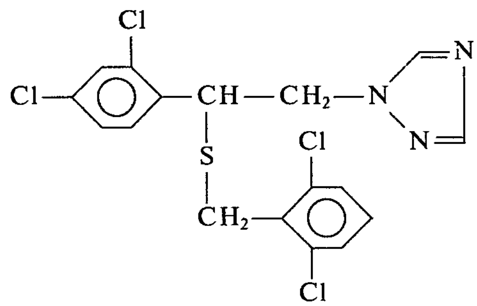 $\times 2HNO_3$ (1) | 0 |

Beispiel B

Podosphaera-Test (Apfel)/protektiv

Lösungsmittel: 4,7 Gewichtsteile Aceton
Emulgator: 0,3 Gewichtsteile Alkylarylpolyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23°C und einer relativen Luftfeuchtigkeit von 70% gebraucht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse gehen aus der nachfolgenden Tabelle hervor:

Tabelle B

Podosphaera-Test/protektiv

| Wirkstoff | Befall in % bei einer Wirkstoff-konzentration von 0,0001% |
|---|---|
| 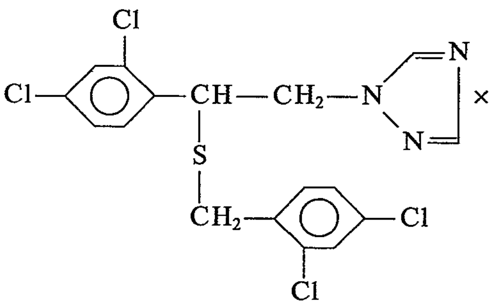 × $HNO_3$<br>(bekannt) | 27 |
| 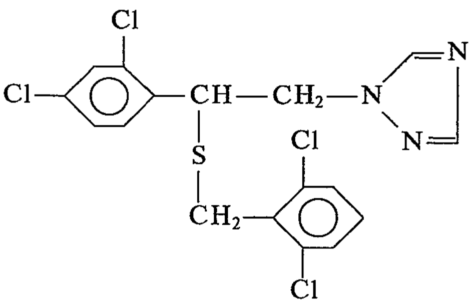 × 2 $HNO_3$<br>(1) | 16 |

Herstellungsbeispiele

Beispiel 1

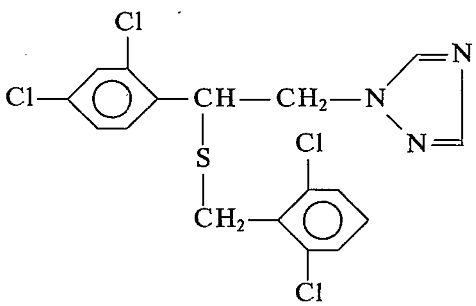 × 2 $HNO_3$

606 g (3,1 Mol) 2,6-Dichlorbenzylchlorid werden in 2,5 l Ethanol gelöst und zu 258 g (3,4 Mol) Thioharnstoff in 1000 ml Ethanol gegeben. Man erhitzt 6 Stunden unter Rühren und Rückfluß, destilliert 1,5 l Ethanol ab, kühlt auf 45°C ab und tropft unter Stickstoffatmosphäre 173,6 g (4,34 Mol) Natriumhydroxid, gelöst in 342 g Wasser, innerhalb von 1,5 Stunden zu. Nach beendeter Zugabe läßt man 30 Minuten bei 45°C nachrühren. Anschließend werden 600 g (2,1 Mol) 1-Chlor-1-(2,4-dichlorphenyl)-2-(1,2,4-triazol-1-yl)-ethan in 2 l Ethanol innerhalb von 3 Stunden zugetropft. Man läßt 15 Stunden bei 40°C nachrühren, destilliert 1,5 l Ethanol bei 50°C im Vakuum ab, läßt abkühlen und versetzt mit 4 l Wasser. Das Reaktionsgemisch wird einmal mit 4 l und dreimal mit je 2 l Methylenchlorid extrahiert; die vereinigten Methylenchloridphasen werden zweimal mit je 2 l Wasser gewaschen, über Natriumsulfat getrocknet und durch Abdestillieren des Lösungsmittels im Wasserstrahlvakuum eingeengt. Der ölige Rückstand wird in 2,5 l Ethanol gelöst und mit 380 g 1,5-Naphthalindisulfonsäure

in 2 l Ethanol versetzt. Der entstandene Niederschlag wird abgesaugt und vorsichtig in 3 l Natriumhydrogencarbonatlösung eingerührt. Die freie Base wird mit 18 l Methylenchlorid extrahiert. Die organische Phase wird durch Abdestillieren des Methylenchlorids eingeengt, der Rückstand in 3 l Chloroform gelöst und tropfenweise mit 66 ml (1,53 Mol) 96%iger Salpetersäure unter Eiskühlung versetzt. Man gibt anschließend 1000 ml Diethylether zu, saugt den entstandenen Niederschlag ab und wäscht mit 500 ml Diethylether. Man erhält 661 g (61,3% der Theorie) 1-(2,4-Dichlorphenyl)-1-(2,6-dichlorbenzylmercapto)-2-(1,2,4-triazol-1-yl)-ethan-nitrat vom Schmelzpunkt 156 – 158° C.

Die obige Verbindung läßt sich auch durch Umsetzung von 1-(2,4-Dichlorphenyl)-1-thiol-2-(1,2,4-triazol-1-yl)-ethan (dessen Herstellung siehe unten) und 2,6-Dichlorbenzylchlorid in Gegenwart eines Verdünnungsmittels (z. B. Ethanol oder Azeton) und einer Base (z. B. Kaliumcarbonat) herstellen.

Vorprodukt:

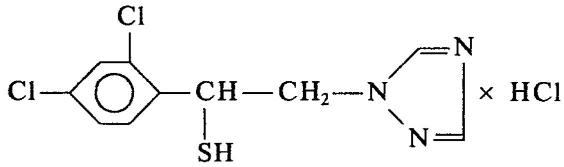

16,05 g (0,05 Mol) 1-Brom-1-(2,4-dichlorphenyl)-2-(1,2,4-triazol-1-yl)-ethan und 4,2 g (0,055 Mol) Thioharnstoff werden in 50 ml Methanol 3 Stunden unter Rückfluß erhitzt. Man läßt auf 0° C abkühlen und tropft unter Stickstoffatmosphäre innerhalb von 30 Minuten 25 ml 2 n-Natronlauge zu. Danach läßt man 2 Stunden bei Raumtemperatur rühren, gibt 100 ml Wasser zu und extrahiert dreimal mit je 50 ml Methylenchlorid. Die vereinigten organischen Phasen werden über Natriumsulfat getrocknet und mit 30 ml etherischer Salzsäure versetzt. Dabei kristallisieren 13,8 g (87,6% der Theorie) 1-(2,4-Dichlorphenyl)-2-(1,2,4-triazol-1-yl)-ethan-1-thiol-hydrochlorid vom Schmelzpunkt 149 – 50° C aus.

Die folgenden Verbindungen der allgemeinen Formel

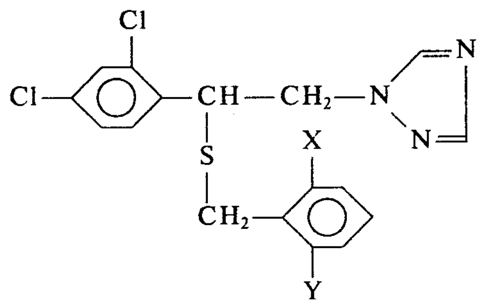

(I)

können in entsprechender Weise erhalten werden:

| Beispiel Nr. | X | Y | Physik. Eigenschaften |
|---|---|---|---|
| 2 | Cl | F | Öl |
| 3 | F | F | Öl |
| 4 | Cl | Cl | $n_D^{20} = 1,6158$ |

**Patentansprüche**

1. 1-(2,4-Dichlorphenyl)-1-(2,6-dihalogenbenzylmercapto)-2-(1,2,4-triazol-1-yl)-ethane der allgemeinen Formel

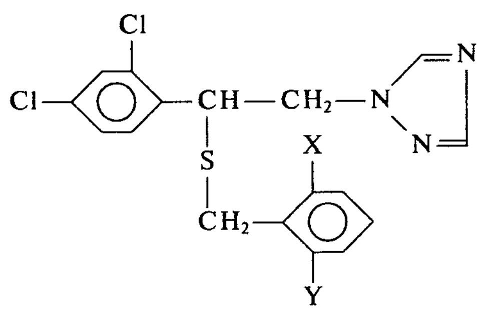

(I)

in welcher

X und Y gleich oder verschieden sind und für Halogen stehen,

und deren physiologisch verträglichen Säureadditions-Salze.

2. Verfahren zur Herstellung von 1-(2,4-Dichlorphenyl)-1-(2,6-dihalogenbenzylmercapto)-2-(1,2,4-triazol-1-yl)-ethanen der allgemeinen Formel

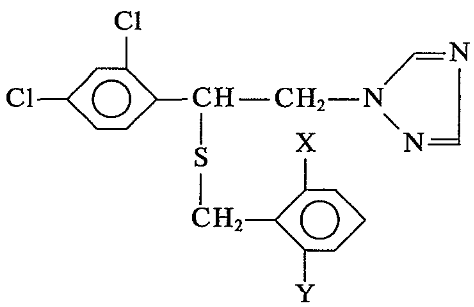

(I)

in welcher

X und Y gleich oder verschieden sind und für Halogen stehen,

und deren physiologisch verträglichen Säureadditions-Salze, dadurch gekennzeichnet, daß man

a) 1-(2,4-Dichlorphenyl)-1-halogen-2-(1,2,4-triazol-1-yl)-ethane der Formel

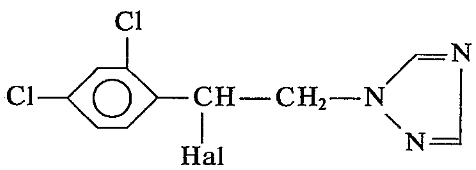

(II)

in welcher

Hal für Halogen steht,

mit Mercaptanen der Formel

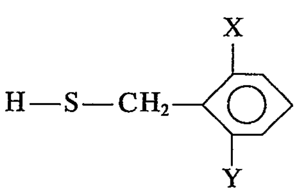

(III)

in welcher

X und Y die oben angegebene Bedeutung haben,

gegebenenfalls in Gegenwart eines Verdünnungsmittels und eines Säurebindemittels umsetzt, wobei die Mercaptane der Formel (III) vorzugsweise in-situ eingesetzt werden oder

b) 1-(2,4-Dichlorphenyl)-2-(1,2,4-triazol-1-yl)-ethan-1-thiol der Formel

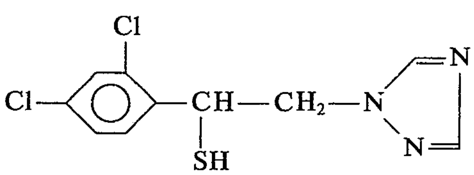

(IV)

mit 2,6-Dihalogenbenzyl-Derivaten der Formel

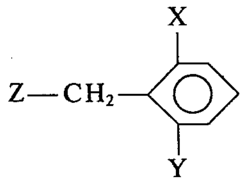

(V)

in welcher

X und Y die oben angegebene Bedeutung haben und
Z für Halogen, und die Gruppierungen $-O-SO_2-R^1$ und $-N(R^2)_3^{\oplus}$ steht, wobei
$R^1$ für Alkyl oder gegebenenfalls substituiertes Phenyl steht und
$R^2$ für Alkyl steht,

in Gegenwart eines Verdünnungsmittels und in Gegenwart einer Base umsetzt, und gegebenenfalls an die so erhaltenen Verbindungen der Formel (I) anschließend eine Säure addiert.

3. Fungizide Mittel, gekennzeichnet durch einen Gehalt an mindestens einem 1-(2,4-Dichlorphenyl)-1-(2,6-dihalogenbenzylmercapto)-2-(1,2,4-triazol-1-yl)-ethan der Formel (I) gemäß Anspruch 1.

4. Verwendung von 1-(2,4-Dichlorphenyl)-1-(2,6-dihalogenbenzylmercapto)-2-(1,2,4-triazol-1-yl)-ethanen der Formel (I) gemäß Anspruch 1, zur Bekämpfung von Pilzen.

5. Verfahren zur Herstellung von fungiziden Mitteln, dadurch gekennzeichnet, daß man 1-(2,4-Dichlorphenyl)-1-(2,6-dihalogenbenzylmercapto)-2-(1,2,4-triazol-1-yl)-ethane der Formel (I) gemäß Anspruch 1 mit Streckmitteln und/oder oberflächenaktiven Mitteln vermischt.

## Claims

1. 1-(2,4-Dichlorophenyl)-1-(2,6-dihalogenobenzylmercapto)-2-(1,2,4-triazol-1-yl)-ethanes of the general formula

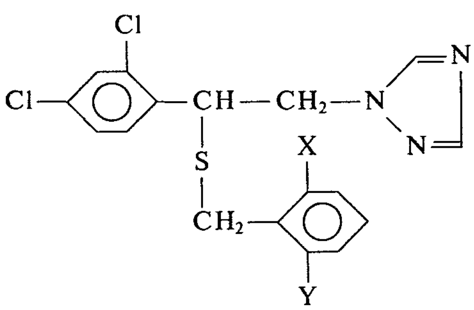

(I)

in which

X and Y are identical or different and represent halogen,

and physiologically acceptable acid addition salts thereof.

2. Process for the preparation of 1-(2,4-dichlorophenyl)-1-(2,6-dihalogenobenzylmercapto)-2-(1,2,4-triazol-1-yl)-ethanes of the general formula

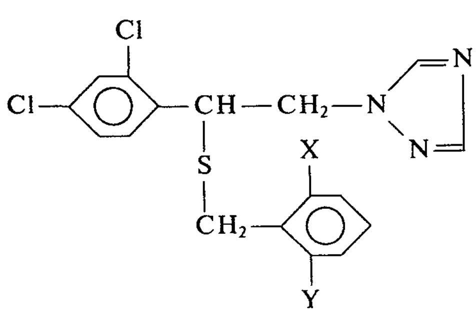

(I)

in which

X and Y are identical or different and represent halogen,

and physiologically acceptable acid addition salts thereof, characterised in that

a) 1-(2,4-dichlorophenyl)-1-halogeno-2-(1,2,4-triazol-1-yl)-ethanes of the formula

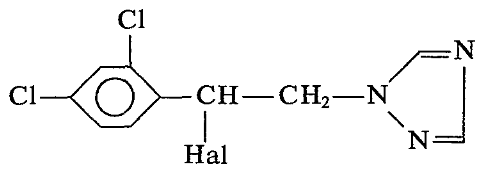

(II)

in which

Hal represents halogen,

are reacted with mercaptans of the formula

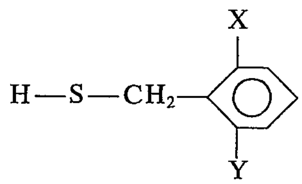

(III)

in which

X and Y have the meaning indicated above,

if appropriate in the presence of a diluent and of an acid-binding agent, the mercaptans of the formula (III) preferably being employed in situ, or

b) 1-(2,4-dichlorophenyl)-2-(1,2,4-triazol-1-yl)-ethane-1-thiol of the formula

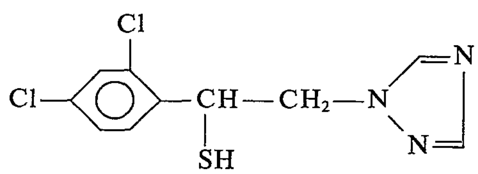

(IV)

is reacted with 2,6-dihalogenbenzyl derivatives of the formula

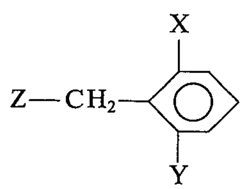

(V)

in which

X and Y have the meaning indicated above and

Z represents halogen or the grouping $-O-SO_2-R^1$ or $-N(R^2)_3^{\oplus}$ wherein

$R^1$ represents alkyl or optionally substituted phenyl and

$R^2$ represents alkyl,

in the presence of a diluent and in the presence of a base, and an acid is then optionally added onto the compounds of the formula (I) thus obtained.

3. Fungicidal agents, characterised in that they contain at least one 1-(2,4-dichlorophenyl)-1-(2,6-dihalogenobenzylmercapto)-2-(1,2,4-triazol-1-yl)-ethane of the formula (I) according to Claim 1.

4. Use of 1-(2,4-dichlorophenyl)-1-(2,6-dihalogenobenzylmercapto)-2-(1,2,4-triazol-1-yl)-ethanes of the formula (I) according to Claim 1 for combating fungi.

5. Process for the preparation of fungicidal agents, characterised in that 1-(2,4-dichlorophenyl)-1-(2,6-dihalogenobenzylmercapto)-2-(1,2,4-triazol-1-yl)-ethanes of the formula (I) according to Claim 1 are mixed with extenders and/or surface-active agents.

**Revendications**

1. 1-(2,4-dichlorophényl)-1-(2,6-dihalogénobenzylmercapto)-2-(1 2,4-triazole-1-yl)-éthanes de formule générale

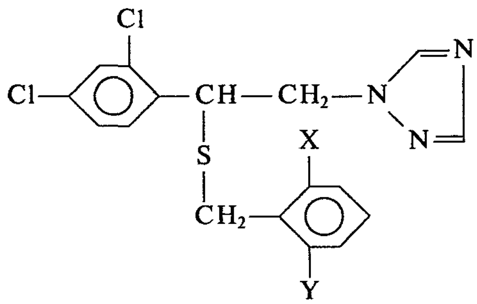

(I)

dans laquelle

X et Y, identiques ou différents, représentent des halogènes,

et leurs sels formés par addition avec des acides tolérés par l'organisme.

2. Procédé de préparation des 1-(2,4-dichlorophényl)-1-(2,6-dihalogénobenzylmercapto)-2-(1,2,4-triazole-1-yl)-éthanes de formule générale

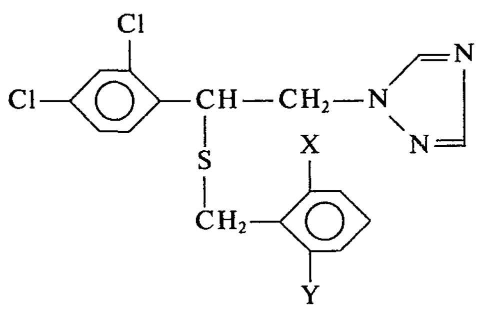

(I)

dans laquelle

X et Y, identiques ou différents, représentent des halogènes,

et de leurs sels formés par addition avec des acides tolérés par l'organisme, caractérisé en ce que:

a) on fait réagir des 1-(2,4-dichlorophényl)-1-halogéno-2-(1,2,4-triazole-1-yl)-éthanes de formule

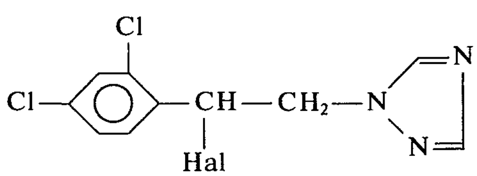

(II)

dans laquelle

Hal représente un halogène

avec des mercaptans de formule

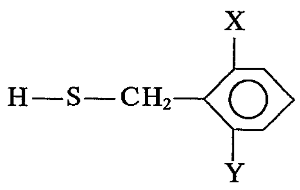

(III)

dans laquelle

X et Y ont les significations indiquées ci-dessus,

éventuellement en présence d'un diluant et d'un agent fixant les acides, les mercaptans de formule III étant de préférence mis en oeuvre in situ, ou bien

b) on fait réagir le 1-(2,4-dichlorophényl)-2-(1,2,4-triazole-1-yl)-éthane-1-thiol de formule

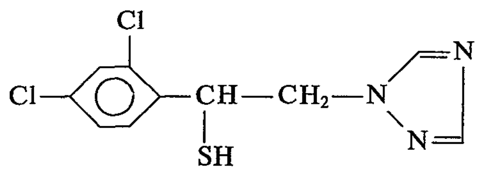

(IV)

avec des dérivés 2,6-dihalogénobenzyliques de formule

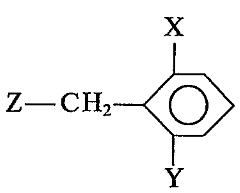

(V)

dans laquelle

X et Y ont les significations indiquées ci-dessus, et

Z représente un halogène, et les groupements $-O-SO_2R^1$ et $-N(R^2)_3^{\oplus}$

$R^1$ représentant un groupe alkyle ou un groupe phényle éventuellement substitué, et

$R^2$ un groupe alkyle,

en présence d'un diluant et en présence d'une base, et le cas échéant, sur les composés de formule I ainsi obtenus, on fixe un acide par addition.

3. Produit fongicide caractérisé en ce qu'il contient au moins un 1-(2,4-dichlorophényl)-1-(2,6-dihalogénobenzylmarcapto)-2-(1,2,4-triazole-1-yl)-éthane de formule I selon la revendication 1.

4. Utilisation des 1-(2,4-dichlorophényl)-1-(2,6-dihalogénobenzylmercapto)-2-(1,2,4-triazole-1-yl)-éthanes de formule I selon la revendication 1 dans la lutte contre les mycètes.

5. Procédé de préparation de produits fongicides, caractérisé en ce que l'on mélange des 1-(2,4-dichlorophényl)-1-(2,6-dihalogénobenzylmercapto)-2-(1,2,4-triazole-1-yl)-éthanes de formule I selon la revendication 1 avec des diluants et/ou des agents tensioactifs.